# FASCICULE DE BREVET EUROPEEN

(11) **EP 0 947 499 B1**
(45) Date de publication et mention de la délivrance du brevet: **20.02.2002**
(21) Numéro de dépôt: 99420077.2
(22) Date de dépôt: 26.03.1999
(51) Int. Cl.: C07C 211/42, C07C 233/14, C07C 209/28

(54) **Procédé de préparation de la sertraline racémique**
Verfahren zur Herstellung von Racemisch Sertralin
Process for the preparation of racemic sertraline

(30) Priorité: 01.04.1998 FR 9804270
(43) Date de publication de la demande: 06.10.1999
(73) Titulaire: Catalys, 69003 Lyon (FR)
(72) Inventeur: Bigot, Patrick, 69008 Lyon (FR)
(74) Mandataire: Guerre, Dominique

(56) Documents cités:
- EP-A- 0 030 081
- WO-A-93/01161
- WO-A-93/01162

## Description

La présente invention a pour objet un procédé de préparation de la cis-N-méthyl-4(3,4-dichlorophényl)-1,2,3,4-tétrahydro-1-naphtylamine racémique, encore dénommée sertraline racémique, répondant à la formule (I) suivante : 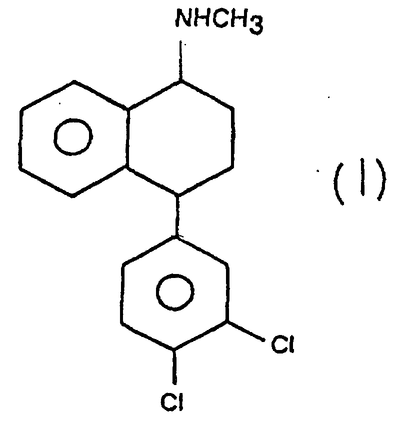 isolé sous forme de sel.

L'énantiomère S,S de la sertraline, sous forme du sel chlorhydrate, répondant à la formule suivante : 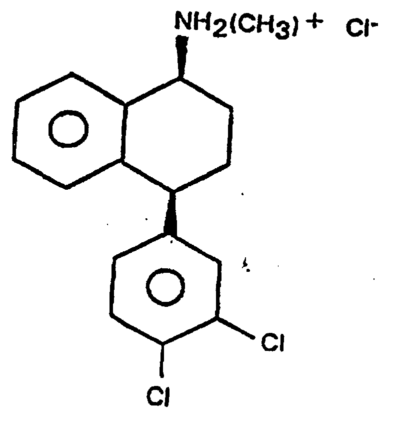 présente une application thérapeutique dans le traitement de la dépression et des problèmes associés, telle que décrite par exemple dans le document EP-0 030 081.

A ce jour, le sel racémique duquel est isolé le sel pharmaceutiquement actif précité, est obtenu à partir de la 4-(3,4-dichlorophényl)-tétralone de formule (II) suivante :

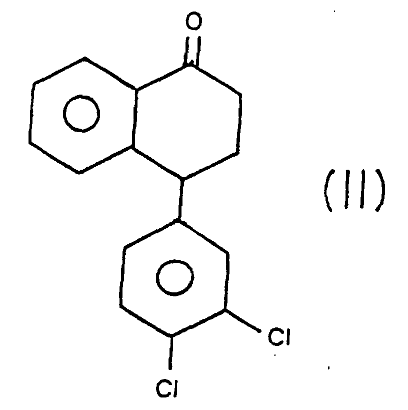

Conformément à la synthèse du composé (I) sous forme de sel décrite dans ledit document EP-0 030 081, on fait réagir la 4-(3,4-dichlorophényl)-tétralone de formule (II) avec la méthylamine anhydre, en présence d'un réactif de déshydratation, on effectue une réaction d'hydrogénation sur charbon palladié du produit de condensation obtenu, et on sépare le sel du cis-N-méthyl-4-(3,4-dichlorophényl)-1,2,3,4-tétrahydro-1-naphtylamine racémique, par cristallisation sélective.

Ce procédé présente plusieurs inconvénients. D'abord il met en jeu des réactifs dangereux tels que la méthylamine anhydre, le tétrachlorure de titane et l'acide chlorhydrique gazeux. Ensuite, au cours de ce procédé, il se produit une hydrogénolyse partielle des substituants chlorés du noyau phényle, conduisant à la formation de composés de la sertraline monochlorés.

La Demanderesse a mis au point un procédé d'obtention de la sertraline racémique à partir de la 4-(3,4-dichlorophényl)-tétralone évitant les problèmes rencontrés ci-dessus.

La présente invention a pour objet un procédé de préparation, en deux étapes, de la cis-N-méthyl-4-(3,4-dichlorophényl)-1,2,3,4-tétrahydro-1-naphtylamine racémique (ou sertraline racémique) répondant à la formule (I), sous forme de sel, ledit procédé comprenant les étapes selon lesquelles :
(a) on fait réagir la 4-(3,4-dichlorophényl)-tétralone de formule (II) ci-dessus décrite, avec le N-méthylformamide en présence d'acide formique,
(b) on traite le milieu réactionnel obtenu selon (a) par une base, et
(c) on sépare le sel du cis-N-méthyl-4-(3,4-dichlorophényl)-1,2,3,4-tétrahydro-1-naphtylamine racémique par cristallisation sélective avec un acide.

Le procédé de l'invention est très avantageux car, à partir du sel auquel il conduit, la sertraline peut être obtenue en une seule étape de cristallisation, à l'état pratiquement pur.

De préférence, à l'étape (a), la 4-(3,4-dichlorophényl)-tétralone de formule (II) est en mélange avec la 4-(2,3-dichlorophényl)-tétralone de formule (III) décrite ci-après.

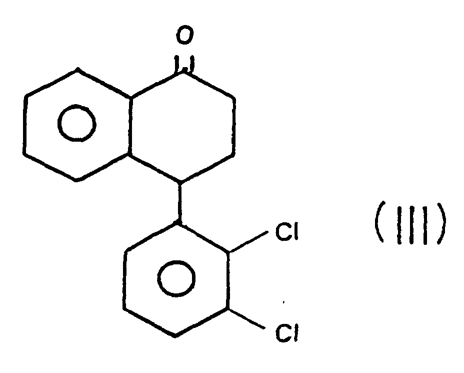

Cette variante du procédé de l'invention est particulièrement avantageuse pour les raisons suivantes :
- la 4-(3,4-dichlorophényl)-tétralone de formule (II) pure est obtenue en cinq étapes conformément au procédé décrit dans le document précité EP-0 030 081 ; sa synthèse en une étape seulement est aussi décrite dans EP-0 346 226, par réaction de l'α-naphtol sur l'orthodichlorobenzène en présence d'un acide de Lewis comme le chlorure d'aluminium ; pour obtenir la tétralone à l'état sensiblement pur, selon cette seconde voie, delx étapes de cristallisation sont nécessaires ; les auteurs de l'article Repinskaya, Sib. Khim. Zh. (1993) 73-76 (Chemical Abstracts, 120, 106497) ont démontré que dans les conditions de l'obtention de la tétralone en une seule étape (EP-0 346 226), on obtient en fait un mélange des isomères 4-(3,4-dichlorophényl)-tétralone de formule (II) et 4-(2,3-dichlorophényl)-tétralone de formule (III) dans un rapport de (II) à (III) de 4,9 / 1 ; la variante du procédé de l'invention propose de faire réagir le mélange de tétralones (II) et (III) directement après réaction de l'α-naphtol sur l'ortho-dichlorobenzène, sans purification préalable de la tétralone (II) ;
- le fait de disposer de la 4-(3,4-dichlorophényl)-tétralone de formule (II) en mélange avec la 4-(2,3-dichlorophényl)-tétralone de formule (III), n'alourdit pas l'étape de séparation du sel à obtenir.

Dans le cadre de l'invention, des mises en oeuvre préférentielles du procédé ont été déterminées ; elles sont ci-après exposées.

Pour l'étape (a), les caractéristiques suivantes peuvent être sélectionnées seules, elles seront avantageusement choisies en combinaison.

L'étape (a) est effectuée à une température variant de 150°C à 220°C, de préférence variant de 180°C à 200°C.

Le N-méthylformamide est présent en un rapport molaire variant de 1,1 à 5 par rapport au mélange des 4-(dichlorophényl)-tétralones de formule (II) et (III), respectivement, et préférentiellement variant de 2,5 à 3.

L'acide formique, qui agit en tant que réducteur dans cette réaction, est avantageusement introduit par fraction de 10 % de la quantité totale à ajouter. Cette dernière représente de préférence un rapport molaire variant de 1,1 à 5, et mieux encore variant de 1,1 à 2, par rapport au mélange précité.

Quand la 4-(3,4-dichlorophényl)-tétralone de formule (II) est en mélange avec la 4-(2,3-dichlorophényl)-tétralone de formule (III), la proportion pondérale de la 4-(3,4-dichlorophényl)-tétralolne dans ce mélange est d'au moins 80 %, celle de la 4-(2,3-dichlorophényl)-tétralone étant au plus de 20 %. Le mélange est très avantageusement obtenu par réaction de l'alpha-naphtol sur l'orthodichlorobenzène, en présence de chlorure d'aluminium, de préférence dans les conditions décrites dans le document EP-0 346 226. Selon les indications décrites dans Chemical Abstracts 120 106497, le mélange ainsi obtenu contient environ 82 % de composé de formule (II) et 18 % de composé de formule (III).

De même pour l'étape (b), il est souhaitable de retenir les caractéristiques suivantes.

La base employée à l'étape (b) pour déformyler les N-méthyl-formamide-4-(dichlorophényl)-1,2,3,4-tétrahydro-1-naphtylamines obtenues à l'étape (a), consiste de préférence en un hydroxyde de métal alcalin, tel que l'hydroxyde de potassium, l'hydroxyde de sodium ou l'hydroxyde de lithium, le premier étant préféré.

Cette base peut être utilisée à l'état pur ou en solution aqueuse.

L'étape (b) est plutôt effectuée dans un solvant hydroxylé, tel qu'un solvant alcoolique. Celui-ci consiste avantageusement en un alcool en C₁-C₈, et de manière préférée il consiste en butanol-1. Des conditions favorables pour la déformylation sont une température de reflux du solvant, et une durée de réaction d'au moins trois heures.

En fin d'étape (b), les formiates alcalins résultants sont dissous dans la phase aqueuse, et le sel de sertraline racémique est obtenu, selon l'étape (c), par cristallisation sélective en traitant la phase organique par un acide, tel qu'un haloacide, de préférence l'acide chlorhydrique, un acide alkylsulfonique, de préférence l'acide méthane-sulfonique, ou un acide arylsulfonique, de préférence l'acide paratoluène-sulfonique.

La précipitation, l'isolement et la purification du sel obtenu à l'étape (c) sont réalisés selon des techniques classiques bien connues de l'homme du métier. Selon l'acide préféré employé, on obtient ainsi un sel choisi parmi le méthane-sulfonate, le paratoluène-sulfonate et le chlorhydrate.

A partir du sel de sertraline racémique, l'homme du métier fera appel à des méthodes connues de séparation d'énantiomères pour obtenir l'énantiomère S,S du chlorhydrate de la sertraline, qui présente des propriétés thérapeutiques. A titre d'exemple, cette séparation peut être réalisée par cristallisation du sel d'un acide optiquement actif ou par chromatographie sur phase chirale suivie d'une salification par l'acide chlorhydrique.

Un autre objet de l'invention est le composé N-méthyl-formamide-4-(3,4-dichlorophényl)-1,2,3,4-tétrahydro-1-naphtylamine, qui répond à la formule (IV) suivante.

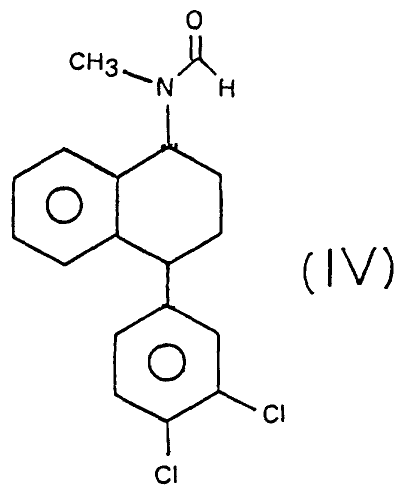

Ce composé constitue un intermédiaire de synthèse de la sertraline. Il peut être obtenu par réaction d'un mélange de 4-(3,4-dichlorophényl)-tétralone de formule (II) et de 4-(2,3-dichlorophényl)-tétralone de formule (III), avec le N-méthylformamide en présence d'acide formique.

L'invention concerne aussi l'utilisation de la N-méthyl-formamide-4-(3,4-dichlorophényl)-1,2,3,4-tétrahydro-1-naphtylamine pour obtenir la cis-N-méthyl-4-(3,4-dichlorophényl)-1,2,3,4-tétrahydro-1-naphtylamine racémique.

Les caractéristiques et avantages de l'invention sont illustrés à l'appui des exemples 1 à 6 suivants. Dans les exemples 1 à 4, la synthèse du sel de sertraline racémique est effectuée à partir d'un mélange des composés de formule (II) et (III), alors que dans les exemples 5 et 6, la synthèse est effectuée à partir du seul composé de formule (II).

### Exemple 1 : obtention du mélange des composés de formule (V) et (VI) à la suite des étapes (a) et (b)

Une suspension de 86,5 g (0,6 moles) d'alpha-naphtol dans 340 ml d'orthodichlorobenzène est mis en réaction avec 160 g de chlorure d'aluminium pendant 2 heures à 120°C. La solution obtenue est hydrolysée par 300 ml d'eau à 80°C. La phase organique inférieure est séparée et l'excès d'orthodichlorobenzène est concentré sous un vide de 20 mm de Hg. Le résidu d'environ 215 g est dissous dans 148 g de N-méthylformamide, et chauffé à 195-200°C pendant 5 heures avec addition fractionnée de 25 g d'acide formique pendant cette période. L'excès de N-méthylformamide est chassé par distillation atmosphérique jusqu'à obtention d'une température de masse de 220°C.

Le mélange réactionnel refroidi à 100°C est dilué par 200 ml de butanol-1 à 100°C. On ajoute 110 g d'hydroxyde de potassium en 1 heure sous agitation et on porte le milieu au reflux pendant 4 heures.

Le milieu est refroidi à 80°C puis dilué par 200 ml d'eau. La phase organique supérieure est séparée, puis traitée comme l'exposent les exemples 2 à 6.

### Exemple 2 : étape (c) par traitement à l'acide méthane-sulfonique

Le mélange d'amines obtenu en solution butanolique selon l'exemple 1, est salifié par 95 g d'une solution aqueuse d'acide méthane-sulfonique à 70 %.

Le précipité formé est filtré, redissous au reflux dans 400 ml de méthanol, traité par 1 g de charbon actif décolorant, filtré et cristallisé entre 0°C et 20°C pour obtenir après filtration et séchage 51,8 g de méthane sulfonate de cis-N-méthyl-(3,4-dichlorophényl)-1,2,3,4-tétrahydro-1-naphtylamine racémique, de point de fusion de 225°C et de pureté par HPLC de 99,7 %.

Le rendement stoechiométrique est de 21,5 % par rapport à l'alpha-naphtol de départ.

### Exemple 3 : étape (c) par traitement à l'acide paratoluène sulfonique

Le mélange d'amines obtenu en solution butanolique selon l'exemple 1, est salifié par 114 g d'acide paratoluène sulfonique monohydraté à 20°C. Le précipité formé est filtré puis redissous dans 800 ml de méthanol au reflux, traité par 1 g de charbon actif décolorant, filtré et cristallisé à 20°C pour obtenir après séchage 65,5 g de paratoluène sulfonate de cis-N-méthyl-(3,4-dichlorophényl)-1,2,3,4-tétrahydro-1-naphtyl-amine racémique, de point de fusion de 280°C et de pureté par HPLC de 97 %.

Le rendement stoechiométrique est de 22 % par rapport à l'alpha-naphtol de départ.

### Exemple 4 : étape (c) par traitement à l'acide chlorhydrique

Le mélange d'amines obtenu en solution butanolique selon l'exemple 1, est salifié par 70 ml d'acide chlorhydrique concentré à 20°C. Le précipité formé est filtré et lavé par 50 ml de butanol-1 pour obtenir 62,1 g de chlorhydrate de cis-N-méthyl-(3,4-dichlorophényl)1,2,3,4-tétrahydro-1-naphtylamine racémique de point de fusion 275-280°C contenant par HPLC 3 % d'isomère cis-N-méthyl-(2,3-dichlorophényl)-1,2,3,4-tétrahydro-1-naphtylamine racémique. Par dissolution dans 620 ml d'éthanol au reflux de ce mélange et cristallisation à 20°C, on obtient un produit contenant 98,5 % d'isomère-3,4 et 1 % d'isomère-2,3. Une recristallisation supplémentaire dans les mêmes conditions permet d'obtenir 48 g de chlorhydrate de cis-N-méthyl-(3,4-dichlorophényl)-1,2,3,4-tétrahydro-1-naphtylamine racémique, de point de fusion de 285-290°C et de pureté par HPLC de 99,5 %. Le rendement stoechiométrique est de 23,3 % par rapport à l'alpha-naphtol de départ.

### Exemple 5 : obtention du composé de formule (V) à la suite des étapes (a) et (b)

58,2 g (0,2 moles) de 4-(3,4-dichlorophényl) tétralone et 49 ml de N-méthylformamide sont chauffés à 200°C, tout en additionnant 10 ml d'acide formique pendant 4 heures.

Le mélange est refroidi, dilué par 80 ml de butanol-1 et 80 ml d'eau. La phase butanolique est séparée puis concentrée sous vide pour obtenir 77 g d'une huile jaune. Une partie aliquote de cette huile est cristallisée dans 40 ml d'éthanol à 0°C pendant 2 jours pour obtenir après filtration et séchage 5,2 g de cristaux blanc-crème (point de fusion 128°C) de cis-N-[4-(3,4-dichlorophényl)-1,2,3,4-tétrahydro-1-naphtyl]-N-méthylformamide. Soit un rendement de 48 % par rapport à la 4-(3,4-dichlorophényl) tétralone de départ.

### Exemple 6 : étape (c) par traitement à l'acide chlorhydrique

La phase butanolique obtenue selon l'exemple 5 est mise en réaction avec 36 g d'hydroxyde de potassium à reflux pendant 2 heures. Après refroidissement, on traite par 100 ml d'eau et on sépare la phase organique supérieure. L'addition sur cette phase de 30 ml d'acide chlorhydrique concentré produit un précipité blanc qui est filtré et séché et donne 28,4 g de chlorhydrate de N-méthyl-4-(3,4-dichlorophényl)-1,2,3,4-tétrahydro-1-naphtylamine de pureté HPLC de 98 % de forme cis et 2 % de forme trans, soit un rendement de 41,4 % par rapport à la 4-(3,4-dichlorophényl)-tétralone de départ.

## Revendications

1. Procédé de préparation de la cis-N-méthyl-4-(3,4-dichlorophényl)-1,2,3,4-tétrahydro-1-naphtylamine racémique répondant à la formule (I), 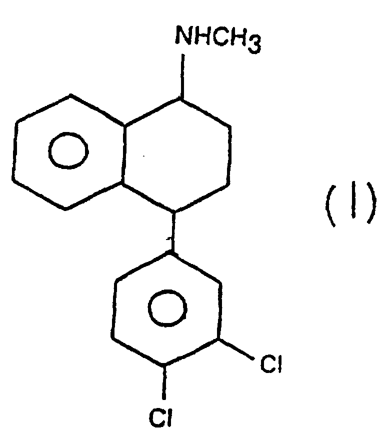 sous forme de sel, à partir de la 4-(3,4-dichlorophényl)-tétralone de formule (II), 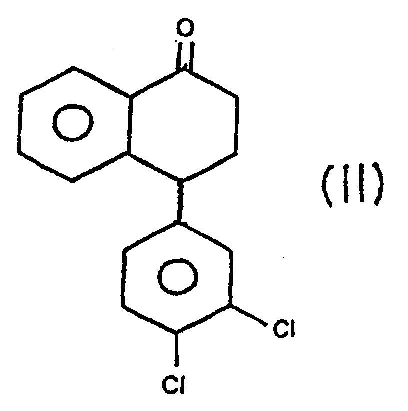 **caractérisé en ce qu'**il comprend les étapes selon lesquelles (a) on fait réagir la 4-(3,4-dichlorophényl)-tétralone de formule (II) avec le N-méthylformamide en présence d'acide formique, (b) on traite le milieu réactionnel obtenu selon (a) par une base, et (c) on sépare le sel de cis-N-méthyl-4-(3,4-dichlorophényl)-1,2,3,4-tétrahydro-1-naphtylamine racémique, par cristallisation sélective avec un acide.

2. Procédé selon la revendication 1, **caractérisé en ce que**, à l'étape (a), la 4-(3,4-dichlorophényl)-tétralone de formule (II) est en mélange avec la 4-(2,3-dichlorophényl)-tétralone de formule (III), 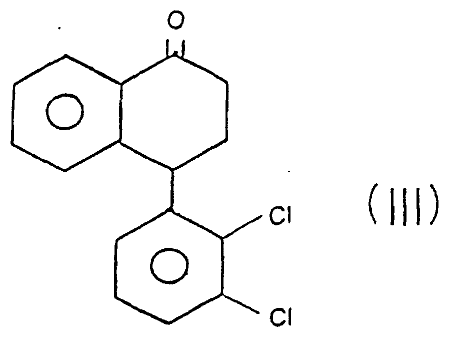 pour obtenir, à titre de composé intermédiaire, le N-méthyl-formamide-4-(3,4-dichlorophényl)-1,2,3,4-tétrahydro-1-naphtylamine de formule (IV) :

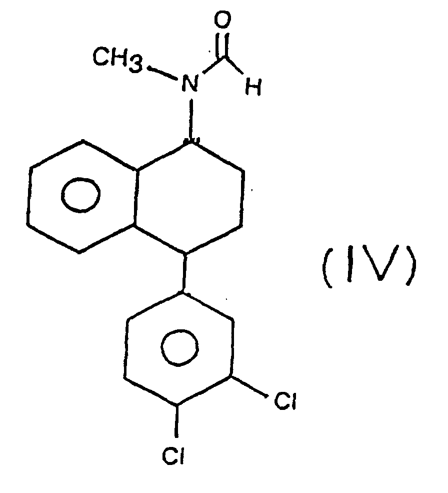

3. Procédé selon la revendication 1 ou 2, **caractérisé en ce que** l'étape (a) est effectuée à une température variant de 150°C à 220°C.

4. Procédé selon la revendication 2, **caractérisé en ce que**, dans le mélange selon l'étape (a), la proportion pondérale de la 4-(3,4-dichlorophényl)-tétralone est d'au moins 80 %, celle de la 4-(2,3-dichlorophényl)-tétralone étant au plus de 20 %.

5. Procédé selon la revendication 4, **caractérisé en ce que** le mélange est obtenu par réaction de l'alpha-naphtol sur l'orthodichlorobenzène, en présence de chlorure d'aluminium.

6. Procédé selon la revendication 1, **caractérisé en ce que** l'étape (b) est effectuée dans un solvant alcoolique en C₁-C₈, porté au reflux.

7. Procédé selon la revendication 6, **caractérisé en ce que** la base est l'hydroxyde de potassium et le solvant est le butanol-1.

8. Procédé selon la revendication 1, **caractérisé en ce que**, selon l'étape (c), la cis-N-méthyl-4-(3,4-dichlorophényl)-1,2,3,4-tétrahydro-1-naphtylamine racémique est obtenu par cristallisation sélective avec un acide choisi parmi l'acide méthane-sulfonique, l'acide paratoluène-sulfonique et l'acide chlorhydrique.

9. Procédé d'obtention du composé intermédiaire, la N-méthyl-formamide-4-(3,4-dichlorophényl)-1,2,3,4-tétrahydro-1-naphtylamine, de formule (IV), obtenu à l'étape (a) du procédé selon la revendication 2, **caractérisé en ce qu'**il comprend l'étape selon laquelle on fait réagir un mélange de 4-(3,4-dichlorophényl)-tétralone de formule (II) et de 4-(2,3-dichlorophényl)-tétralone de formule (III), avec le N-méthylformamide en présence d'acide formique.

## Patentansprüche

1. Verfahren zur Herstellung von cis-N-Methyl-4-(3,4-dichlorophenyl)-1,2,3,4-tetrahydro-1-naphtylamin mit der Formel (I) 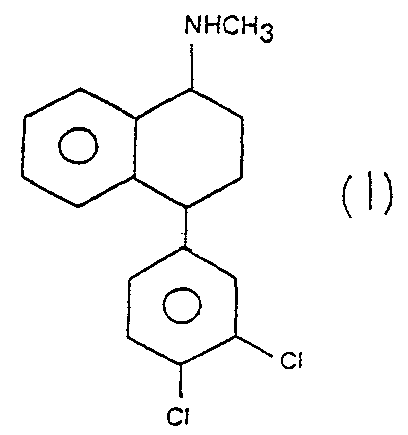 in Salzform, ausgehend von 4-(3,4-Dichlorophenyl)-tetralon der Formel (II), 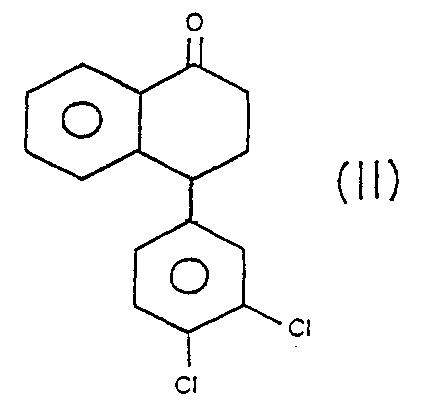 **dadurch gekennzeichnet, daß** es folgende Schritte aufweist, nämlich (a) Reagierenlassen von 4-(3,4-Dichlorophenyl)-tetralon der Formel (II) mit N-Methylformamid in der Gegenwart von Ameisensäure, (b) Behandeln des erhaltenen Reaktionsmilieus von Schritt (a) mit einer Base und (c) Abtrennen des racemischen Salzes von cis-N-Methyl-4-(3,4-dichlorophenyl)-1,2,3,4-tetrahydro-1-naphtylamin durch selektive Kristallisation mit einer Säure.

2. Verfahren nach Anspruch 1, **dadurch gekennzeichnet, daß** in Schritt (a) das 4-(3,4-Dichlorophenyl)-tetralon der Formel (II) sich in Mischung mit dem 4-(2,3-Dichllrophenyl)-tetralon der Formel (III) befindet, 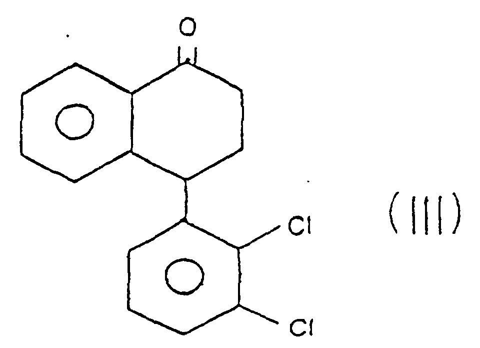 um als intermediäre Verbindung das N-Methylformamid-4-(3,4-dichlorophenyl)-1,2,3,4-tetrahydro-1-naphtylamin der Formel (IV) 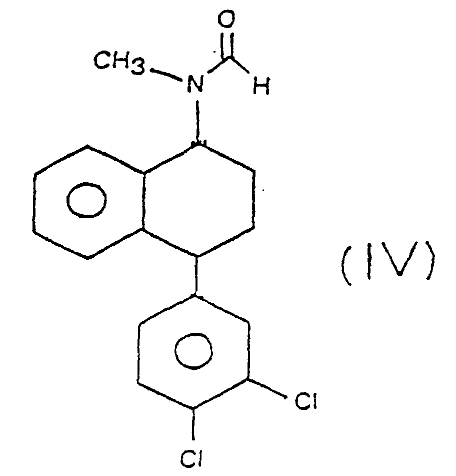 zu erhalten.

3. Verfahren nach Anspruch 1 oder 2, **dadurch gekennzeichnet, daß** der Schritt (a) bei einer Temperatur, die zwischen 150°C und 220°C variiert, durchgeführt wird.

4. Verfahren nach Anspruch 2, **dadurch gekennzeichnet, daß** in der Mischung von Schritt (a) der Gewichtsanteil des 4-(3,4-Dichlorophenyl)-tetralons zumindest 80 % ist, und daß der Gehalt des 4-(2,3-Dichlorophenyl)-tetralons höchstens 20 % beträgt.

5. Verfahren nach Anspruch 4, **dadurch gekennzeichnet, daß** die Mischung durch Reaktion von α-Naphthol mit ortho-Dichlorbenzol in Gegenwart von Aluminiumchlorid erhalten wird.

6. Verfahren nach Anspruch 1, **dadurch gekennzeichnet, daß** der Schritt (b) in einem alkoholischen C₁-C₈ Lösungsmittel durchgeführt wird, das unter Rückfluß gehalten wird.

7. Verfahren nach Anspruch 6, **dadurch gekennzeichnet, daß** die Base Kaliumhydroxid ist, und daß das Lösungsmittel Butanol-1 ist.

8. Verfahren nach Anspruch 1, **dadurch gekennzeichnet, daß** in Schritt (c) das racemische cis-N-Methyl-4-(3,4-dichlorophenyl)-1,2,3,4-tetrahydro-1-naphtylamin durch selektive Kristallisation mit einer Säure ausgewählt aus Methansulfonsäure, para-Toluolsulfonsäure und Chlorwasserstoffsäure erhalten wird.

9. Verfahren zur Herstellung einer intermediären Verbindung, nämlich dem N-Methylformamid-4-(3,4-dichlorophenyl)-1,2,3,4-tetrahydro-1-naphtylamin der Formel (IV), das in Schritt (a) des Verfahrens nach Anspruch 2 erhalten wird, **dadurch gekennzeichnet, daß** es folgenden Schritt aufweist, nämlich Reagierenlassen einer Mischung von 4-(3,4-Dichlorophenyl)-tetralon der Formel (II) und dem 4-(2,3-Dichlorophenyl)-tetralon der Formel (III) mit N-Methylformamid in Gegenwart von Ameisensäure.

## Claims

1. Process for the preparation of racemic cis-(N-methyl-4-(3,4-dichlorophenyl)-1,2,3,4-tetrahydro-1-naphthylamine corresponding to formula (I) 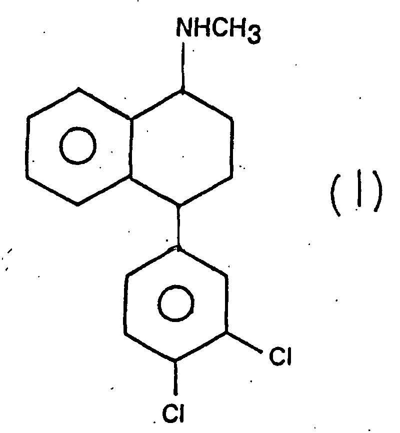 in salt form, starting with 4-(3,4-dichlorophenyl)tetralone of formula (II) 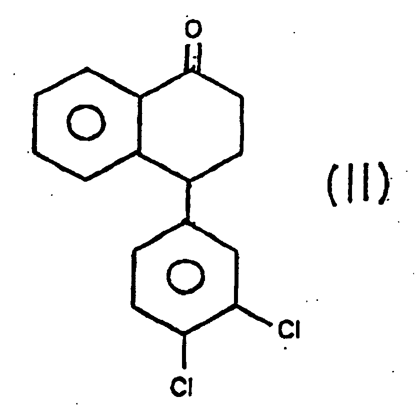 **characterized in that** it comprises the steps according to which (a) 4-(3,4-dichlorophenyl)tetralone of formula (II) is reacted with N-methylformamide in the presence of formic acid, (b) the reaction medium obtained according to (a) is treated with a base, and (c) the salt of racemic cis-N-methyl-4-(3,4-dichlorophenyl)-1,2,3,4-tetrahydro-1-naphthylamine is separated out by selective crystallization with an acid.

2. Process according to Claim 1, **characterized in that**, in step (a), the 4-(3,4-dichlorophenyl)tetralone of formula (II) is in a mixture with 4-(2,3-dichlorophenyl)tetralone of formula (III) 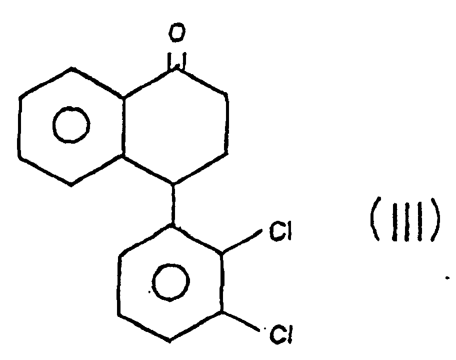 to give, as an intermediate compound, N-methylformamide-4-(3,4-dichlorophenyl)-1,2,3,4-tetrahydro-1-naphthylamine of formula (IV):

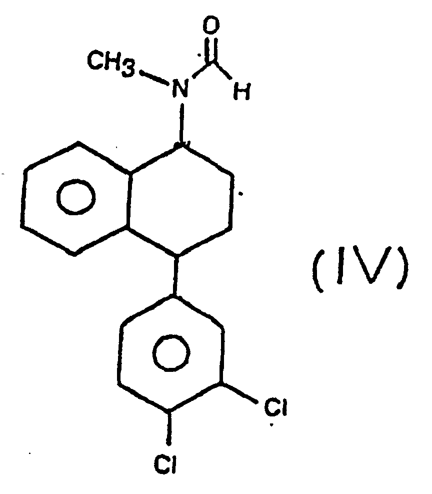

3. Process according to Claim 1 or 2, **characterized in that** step (a) is carried out at a temperature ranging from 150°C to 220°C.

4. Process according to Claim 2, **characterized in that**, in the mixture according to step (a), the weight proportion of the 4-(3,4-dichlorophenyl)tetralone is at least 80%, that of the 4-(2,3-dichlorophenyl) tetralone being not more than 20%.

5. Process according to Claim 4, **characterized in that** the mixture is obtained by reaction of α-naphthol with ortho-dichlorobenzene, in the presence of aluminum chloride.

6. Process according to Claim 1, **characterized in that** step (b) is carried out in a C₁-C₈ alcoholic solvent maintained at reflux.

7. Process according to Claim 6, **characterized in that** the base is potassium hydroxide and the solvent is 1-butanol.

8. Process according to Claim 1, **characterized in that**, according to step (c), the racemic cis-N-methyl-4-(3,4-dichlorophenyl)-1,2,3,4-tetrahydro-1-naphthylamine is obtained by selective crystallization with an acid chosen from methanesulfonic acid, paratoluenesulfonic acid and hydrochloric acid.

9. Process for obtaining the intermediate compound, N-methylformamide-4-(3,4-dichlorophenyl)-1,2,3,4-tetrahydro-1-naphthylamine, of formula (IV), obtained in step (a) of the process according to Claim 2, **characterized in that** it comprises the step according to which a mixture of 4-(3,4-dichlorophenyl) tetralone of formula (II) and 4-(2,3-dichlorophenyl)tetralone of formula (III) is reacted with N-methylformamide in the presence of formic acid.
